# EUROPEAN PATENT SPECIFICATION

(11) **EP 4 200 469 B1**
(45) Date of publication and mention of the grant of the patent: **20.11.2024**
(21) Application number: 21765688.3
(22) Date of filing: 23.08.2021
(51) Int. Cl.: D04B 1/12

(54) **ARTICLE AND METHOD OF MAKING THE SAME**
EIN ARTIKEL UND SEIN HERSTELLUNGSVERFAHREN
UN ARTICLE ET SON PROCÉDÉ DE RÉALISATION

(30) Priority: 24.08.2020 GB 202013174
(43) Date of publication of application: 28.06.2023
(73) Proprietor: Prevayl Innovations Limited, Wilmslow SK9 1LD (GB)
(72) Inventor: RIAZ, Naeem, Manchester, Greater Manchester M2 3AZ (GB)
(74) Representative: Appleyard Lees IP LLP
(86) International application number: PCT/GB2021/052191
(87) International publication number: WO 2022/043672

(56) References cited:
- WO-A1-2014/188171
- WO-A1-2017/044313
- KR-A- 20180 092 685
- US-A1- 2015 164 420

## Description

The present invention is directed towards an article and a method of making the same. The present invention is in particular directed towards articles comprising textile bodies and raised conductive regions.

### Background

Wearable articles can be designed to interface with a wearer of the article, and to determine information such as the wearer's heart rate, rate of respiration, activity level, and body positioning. Such properties can be measured with a sensor assembly that includes a sensor such as an electrode for signal transduction and/or microprocessors for analysis. The articles include electrically conductive pathways to allow for signal transmission between a removable electronics module for processing and communication and sensing components of the article. The wearable articles may be garments. Such garments are commonly referred to as `smart clothing' and may also be referred to as 'biosensing garments' if they measure biosignals.

It is desirable for electrodes of the wearable article to have a raised, three-dimensional profile. Such three-dimensional electrodes provide improved signal quality when measuring signals from the body of the wearer as the skin contact with the electrode is generally improved and is more robust against wearer motion than flat electrodes.

US Patent Application Publication No. US2012144561 A1 discloses a three-dimensional textile electrode. The electrode is knit using conducting thread while a base fabric is knit using nonconducting thread. The electrode is knit on a first needle bed and the base fabric is knit on a second needle bed opposite to and facing the first needle bed, the two needle beds being separated by a few millimetres. During the knitting process, the surface knit on the first needle bed and the surface knit on the second needle bed may be linked using an isolating thread network that is simply deposited, without forming a mesh, on the fabric, in order to provide the three-dimensional effect.

US Patent Application Publication No. US20150164420 discloses a three-dimensional electrically conductive fabric that includes a resilient conductive tissue, a foundation tissue, and a support tissue. The support tissue is arranged between and connects the resilient conductive tissue and the foundation tissue. The resilient conductive tissue, the foundation tissue, and the support tissue are unitarily combined through knitting to form the structure of three-dimensional electrically conductive fabric.

International Patent Application Publication No. WO2014188171 discloses a contact sensor for incorporation within clothing to monitor activity at a body surface. The sensor includes a contact membrane having a body surface contacting area and one or more base layers of knitted fabric. The base layer(s) is thicker over an area congruent with the body surface contacting area of the contact membrane. As a result, the contact membrane is urged into the forming of a raised outer surface for projection against a body surface.

International Patent Application Publication No. WO2017044313 discloses a method of manufacturing an article of footwear that includes providing a textile and applying heat and/or pressure to the textile using a texturing device to form a textured area of the textile. The textured area is spaced apart from a substantially smooth area of the textile.

It is desirable to provide an improved approach for forming raised conductive regions on a textile.

### Summary

According to the present disclosure there is provided an article and method as set forth in the appended claims. Other features of the invention will be apparent from the dependent claims, and the description which follows.

According to a first aspect of the disclosure, there is provided an article comprising a textile body, a conductive region that forms an electrode arranged to measure or apply signals to a further object, and an embossing material. The embossing material causes the conductive region to adopt and retain a raised, embossed, profile that projects outwardly from a surface of the textile body, and comprises a heat and/or pressure-activated adhesive material.

Advantageously, the present disclosure provides an article with a conductive region that forms a raised profile extending away from a surface of the textile body. This three-dimensional conductive region may form a raised electrode, for example. The conductive region is an embossed conductive region formed using embossing techniques. In particular an embossing material is used to cause the conductive region to adopt and (permanently or near permanently) retain the raised, embossed, profile even after repeated use and/or washing of the article. An embossing material will be understood as referring to a material which under the application of heat and/or pressure or similar stimulus will cause the conductive region to adopt and retain the raised, embossed, profile. The embossing material is a material that can be moulded to a desired shape and bonded to the textile body by the application of a stimulus and then maintains the moulded shape following the removal of the stimulus. Embossing materials are known in the art. The embossing material may refer to a material that is fusible, bondable, adhesive, or thermoformable. The embossing material may have a melting point of between 50 degrees Centigrade and 250 degrees Centigrade but is not limited to this particular melting point range.

The use of an embossing material means that an isolating thread network, filler yarn or other filler material are not required to be densely packed into the space between a conductive region and the textile body to cause the conductive region to adopt the raised profile. In addition, a variable thickness of the textile body is not required. The textile body can have a uniform thickness. The article of the present disclosure has a simpler, less specialised, manufacturing process while still providing the desired raised conductive region in the article. While a filler material is not required, a filler material may also still be used if desired by the skilled person. Established embossing techniques can be used to form the raised conductive profile.

The embossing material may form an embossing layer.

The embossing material may be applied to a surface of the textile body such as to form an embossing layer. The embossing material may be applied to a surface of the textile body which opposes a surface on which the conductive region is located. The embossing material and the textile body may thus be provided on opposing surfaces of the textile body. The embossing material may be aligned with the conductive region. The embossing material may be aligned with the conductive region but spatially separated from the conductive region by a portion of the textile body. The textile body may thus be provided between the conductive region and the embossing material. The embossing material may cover a larger area of the textile body than the conductive region. The embossing material may cover a smaller area of the textile body than the conductive region. The embossing material may be provided in a plurality of discrete regions such as to cover part of the area covered by the conductive region. Providing the embossing material in a limited number of discrete regions may beneficially provide the desired raised profile while still imparting a high-degree of flexibility to the article. The embossing material may be provided between the textile body and the conductive region.

The embossing material may comprise an embossing yarn. The embossing yarn may be knitted, woven, embroidered, braided, felted or otherwise attached to the textile body. The embossing yarn may be located between the textile body and the conductive region. The embossing yarn is a yarn that is able to be moulded to a desired shape and subsequently retain (permanently or near permanently) its desired shape. This means that the space between the textile body and the conductive yarn does not need to be densely packed with an isolating thread network to form the raised profile. Instead, the embossing yarn is provided which is moulded to form the desired shape. The embossing yarn may refer to a fusible bonding yarn. The fusible bonding yarn may be referred as an adhesive or thermoformable yarn. The embossing yarn may be a thermal fusion bonding (TFB) yarn. The embossing yarns may comprise nylon, polyester, or other suitable thermoformable yarn material. The embossing may be the GRILON (RTM) fusible bonding yarn marketed by DISTRICO, 9 rue Mayran, 75009 Paris France. Advantageously, when using an embossing yarn, the textile body, conductive region, and embossing material can be formed from a single piece of (e.g. knitted or woven) fabric. That is, the different portions of the article can be integrally knit or woven together during a single knitting or weaving operation.

The embossing material comprises an adhesive material. The embossing material may be able to be adhered to the textile body such as upon the application of heat and/or pressure. The embossing material may be modulable such that it may adopt the shape of a mould or tool of an embossing machine that applies the heat and/or pressure to the article. The embossing material may be able to retain the shape of the tool/mould following the removal of the heat and/or pressure such that the conductive region permanently or near permanently retains the three-dimensional profile. The embossing material may have waterproof properties such that it is impervious or near impervious to water. This may beneficially help prevent the ingress of water into the textile body.

The adhesive material may comprise a heat-activated adhesive material which may also be referred to as a heat-sensitive adhesive material or a hot-melt adhesive material. A heat-activated adhesive material generally refers to a material that will not bond at normal temperatures (e.g. room temperature) but will bond under the application of heat. Such heat-activated adhesive materials can be used with heat presses and other forms of embossing machines to cause them to melt and bond with the textile body and cause the conductive region to adopt the raised, embossed, profile. Upon cessation of the application of heat, the heat-activated adhesive will harden can cause the conductive region to retain the raised, embossed, profile.

The adhesive material may comprise a pressure-activated adhesive material which may also be referred to as a pressure-sensitive adhesive material. A pressure-activated adhesive material generally refers to a material that will not bond at low pressures (e.g. atmospheric pressures) but will bond under the application of pressure. Such pressure-activated adhesive materials can be used with heat presses, rollers, and other forms of embossing machines to cause them to melt and bond with the textile body and cause the conductive region to adopt the raised, embossed, profile. Upon cessation of the application of pressure, the pressure-activated adhesive will harden can cause the conductive region to retain the raised, embossed, profile. The adhesive material may comprise a combination of heat-activated and pressure-activated adhesive material or may comprise adhesive materials which are both heat-activated and pressure-activated.

The embossing material may comprise silicone. The embossing material may comprise nylon or polyester and, in particular, low-melt nylon or polyester. The embossing material may be any form of thermoformable material.

The embossing material may be applied to a surface of the textile body in a liquid form such as in the form of an ink. The embossing material may thus be printed onto the textile body such as by screen printing, inkjet printing or other known printing methods. The ink may comprise a silicone ink. The ink may comprise a catalyst to activate the silicone. The ink may thus be a mixture of silicone ink and a catalyst. Such inks are known for use in embossing materials.

The embossing material may comprise a film of material that is applied to a surface of the textile body. The film of material may be referred to as an embossing film. The embossing film may be an adhesive film that may be adhered to the textile body such as following the application of heat and/or pressure. The embossing film may be heat bonded to the textile body. The embossing film may comprise silicone. The silicone embossing film is able to be heat bonded, is adhesive, mouldable and waterproof. The silicone embossing film is able to retain the shape of the tool/mould of an embossing machine used to apply the embossing film to the textile body.

Applying the embossing material to a surface of the textile body such as in the form of an ink or film beneficially means that specialist knitting techniques / yarns are not required to form the embossing region. Instead, established printing techniques or the simple act of positioning a film on the textile body are used. While these techniques are simpler, they do require a separate process to be performed which may increase the manufacture time. The use of an embossing yarn (e.g. a thermal fusion bonding yarn) is also in the scope of the present disclosure. The embossing yarn may be integrally knit or woven with the textile body/conductive region during a single knitting operation which means that multiple different processes are not required. It will be appreciated that both techniques achieve their own advantages and may be used in different situations depending on, for example, the equipment and personnel at the manufacturing location.

The textile body may be any form of textile body and is generally preferred to be non-conductive or a least comprise non-conductive regions. The textile body may be made using any textile construction techniques known in the art such as knitting, weaving or felting. The textile body may comprise one or more types of yarn preferably non-conductive yarn. The textile body may comprise a base yarn and one or more additional yarns may be provided so as to add stretch to the textile body. The one or more additional yarns may be elastomeric yarns. In preferred examples, the textile body is a knitted component and in particular a weft knitted component.

The conductive region may comprise a conductive material that is applied to the textile body. The conductive material may be in the form of a conductive ink that is printed onto the textile body such as by using screen printing or inkjet printing techniques. The conductive region may be provided in the form of a transfer that is adhered to the textile body. The transfer may comprise one or more cured conductive ink layers that may be separated by cured non-conductive ink layers. An adhesive layer of the transfer may enable the transfer to be adhered to the textile body such as under the application of heat and/or pressure.

In preferred examples, the conductive region comprises a conductive textile. The conductive textile may be a knitted, woven, felted or embroidered. The conductive region may comprise conductive yarn. The conductive region may be attached to the textile body such as by being stitched or adhered to the textile body. In preferred examples still, the conductive region is integrally formed with the textile body such as during a single knitting, weaving or felting operation. In most preferred examples, the conductive region is a knitted component and in particular a weft knitted component that is formed integrally with a corresponding weft knitted textile body. The conductive region may be knitted from a single length of conductive yarn.

The conductive region may form a connection region for forming a conductive connection with a further object. The conductive region forms an electrode and is able to measure or apply signals to a further object.

The article may comprise a plurality of embossed conductive regions. At least two embossed conductive regions may be provided on opposing surfaces of the textile body. This may mean that embossing material is applied to the opposing surfaces of the textile body.

A conductive pathway may extend between at least two of the embossed conductive regions. Embossing material may cover at least part of the conductive pathway. The conductive pathway may not have a three-dimensional profile. That is, the embossing material covering the at least part of the conductive pathway may not be moulded to form a three-dimensional shape. Advantageously, the embossing material can insulate/waterproof the conductive pathway. This step can be performed at the same time as applying the embossing material to a conductive region (e.g. the same embossing film can be used). This simplifies the manufacturing process and means that a separate insulating/waterproofing step may not be required. Embossing material may not cover at least part of the conductive pathway.

The article may form or may be part of a wearable article. The wearable article may form or be part of a garment.

According to a second aspect of the disclosure, there is provided a method of forming a raised profile in a conductive region of an article. The conductive region forms an electrode arranged to measure or apply signals to a further object.

The method comprises applying heat and/or pressure to an article to cause the article to adopt a raised, embossed, profile that projects outwardly from a surface of a textile body of the article. The raised, embossed, profile is retained upon release of the applied heat and/or pressure due to an embossing material of the article bonding to the textile body following the application of heat and/or pressure. The raised, embossed, profile is a conductive region of the article.

In some examples, the conductive material may be applied to the textile body following the application of heat and/or pressure to the article. That is, the conductive material may be applied to already formed embossed regions of the article. Generally, however, it is preferred to form the article comprising the textile body, conductive region, and embossing material first prior to applying the heat and/or pressure. The method may comprise providing an article comprising the textile body, conductive region, and embossing material. The heat and/or pressure may be applied to the article comprising the textile body, conductive region and embossing material.

The method may comprise providing an article comprising a textile body and optionally a conductive region. The method may comprise applying embossing material to the textile body of the article. The embossing material may be printed onto the textile body of the article. The embossing material may be provided as a film of embossing material that is positioned on the textile body of the article. Providing the article comprising the textile body and optionally conductive region may comprise knitting or weaving the textile body and optionally conductive region.

The method may comprise providing an article comprising a textile body, embossing material, and optionally a conductive region. The embossing material may comprise an embossing yarn. Providing the article may comprise knitting or weaving the textile body, embossing material and optionally conductive region.

Applying heat and/or pressure to the article may comprise providing a tool; and using the tool to apply pressure to the article to cause the article to adopt the raised, embossed, profile. Applying heat and/or pressure to the article may further comprise providing a mould component having a cavity and may comprise using the tool to apply pressure to the article to distort the article into the cavity of the mould component so as to adopt the raised profile.

The tool may have a structured surface. The structure surface may comprise a positive profile that corresponds to the raised profile to be formed in the article or a negative profile that is the inverse of the raised profile to be formed in the article.

According to a non-claimed aspect of the disclosure, there is provided a system comprising the article of the first aspect of the disclosure and an electronics module arranged to form a communicative coupling with the article.

The present disclosure is not limited to wearable articles. The articles may include upholstery, such as upholstery that may be positioned on pieces of furniture, vehicle seating, as wall or ceiling décor, among other examples.

### Brief Description of the Drawings

Examples of the present disclosure will now be described with reference to the accompanying drawings, in which:
Figures 1 to 6 show a sequence of steps involved in forming an article according to the invention.
Figure 7 shows a process flow diagram for an example method of forming a raised conductive region on an article according to the invention.
Figure 8 shows a process flow diagram for another example method of forming a raised conductive region on an article according to the invention.
Figure 9 shows a process flow diagram for another example method of forming a raised conductive region on an article according to the invention.
Figure 10 shows a process flow diagram for another example method of forming a raised conductive region on an article according to the invention.
Figure 11 shows a side view of an example article according to the invention.
Figure 12 shows a bottom view of the article of Figure 11;
Figure 13 shows a top view of the article of Figure 11;
Figure 14 shows a top view of an example article according to the invention.
Figure 15 shows an electronics module positioned on a surface of the article of Figure 14;
Figure 16 shows a bottom view of the article of Figure 14;
Figure 17 shows a side view of the article of Figure 14 with the electronics module positioned on the first surface of the wearable article;
Figure 18 shows a schematic diagram for an example system according to aspects of the present disclosure; and
Figure 19 shows a schematic diagram for an example electronics module according to aspects of the present disclosure.

### Detailed Description

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope of the independent claims. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the invention as defined by the appended claims and their equivalents.

It is to be understood that the singular forms "a," "an," and "the" include plural referents unless the context clearly dictates otherwise.

"Wearable article" as referred to throughout the present disclosure may refer to any form of electronic device which may be worn by a user such as a smart watch, necklace, bracelet, headphones, in-ear headphones, or glasses. The wearable article may be a textile article. The wearable article may be a garment. The garment may refer to an item of clothing or apparel. The garment may be a top. The top may be a shirt, t-shirt, blouse, sweater, jacket/coat, or vest. The garment may be a dress, brassiere, shorts, pants, arm or leg sleeve, vest, jacket/coat, glove, armband, underwear, headband, hat/cap, collar, wristband, stocking, sock, or shoe, athletic clothing, swimwear, personal protection equipment, wetsuit or drysuit.

The wearable article/garment may be constructed from a woven or a non-woven material. The wearable article/garment may be constructed from natural fibres, synthetic fibres, or a natural fibre blended with one or more other materials which can be natural or synthetic. The yarn may be cotton. The cotton may be blended with polyester and/or viscose and/or polyamide according to the particular application. Silk may also be used as the natural fibre. Cellulose, wool, hemp and jute are also natural fibres that may be used in the wearable article/garment. Polyester, polycotton, nylon and viscose are synthetic fibres that may be used in the wearable article/garment.

The garment may be a tight-fitting garment. Beneficially, a tight-fitting garment helps ensure that the sensor devices of the garment are held in contact with or in the proximity of a skin surface of the wearer. The garment may be a compression garment. The garment may be an athletic garment such as an elastomeric athletic garment. The present disclosure is not limited to wearable articles for humans and includes wearable articles for animals such as animal collars, jackets and sleeves.

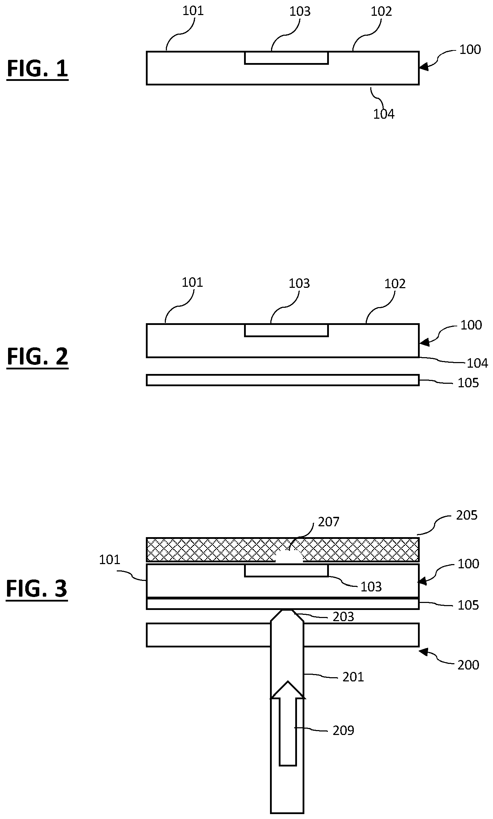

Referring to Figures 1 to 6, there is shown a series of steps involved in forming a raised conductive region on an article 100.

Figure 1 shows that a component of the article 100 is provided. The component of the article 100 comprises a textile body 101 and a conductive region 103. The conductive region 103 is provided on a first surface 102 of the textile body 101. The conductive region 103 is substantially flat and does not substantially extend away from the surface of the textile body 101. In some examples, the conductive region 103 may extend, to an extent, away from the textile body 101 but is generally not able to retain its shape. The conductive region 103 may be flush with or may be recessed within the first surface 102 of the textile body 101.

The textile body 101 may be any form of textile, e.g. fabric, body 101. The textile body 101 is generally knitted or woven from non-conductive yarn. In preferred examples, the textile body 101 is knitted from one or more types of non-conductive yarn using a weft knitting process.

The conductive region 103 may be any form of conductive region 103. Preferably, the conductive region is conductive textile, e.g. a conductive fabric region 103. The conductive region 103 may be knitted or woven from non-conductive yarn and may be formed with the textile body 101 during the same knitting/weaving procedure. In this example, the conductive region 103 is integrally knit with the textile body 101 using a weft knitting process.

Figure 2 shows that an embossing material 105 is applied to a second surface 104 of the textile body 101 that opposes the first surface 102. The embossing material 105 is aligned with / coincident with the conductive region 103 and spatially separated from the conductive region 103 by the textile body 101. The textile body 101 is provided between the embossing material 105 and the conductive region 103. The embossing material 105 covers an area of the second surface 104 that is greater than an area covered by the conductive region 103 on the first surface 102. Providing an embossing material region 105 that is larger than the conductive region 103 is beneficial as the embossing material 105 typically has waterproofing properties. In this way, the embossing material 105 can be used to waterproof at least part of the textile body 101.

The embossing material 105 in the example of Figure 2 is provided in the form of a heat and/or pressure activated adhesive film comprising silicone. The embossing film 105 is positioned on the second surface 104 of the textile body 101. Subsequent application of heat and/or pressure causes the embossing film 105 to bond to the textile body 101.

Figure 3 shows that the article 100 comprising the textile body 101, conductive region 103, and embossing material 105 is positioned within an embossing machine 200. The embossing machine 200 in this example applies pressure and/or heat to cause the conductive region 103 to adopt a raised, embossed, profile. The pressure and/or heat activates the embossing material 105 and causes it to bond to the textile body 101 such that the conductive region 103 retains the embossed profile following the removal of the applied pressure and/or heat.

The embossing machine 200 shown in Figure 3 comprises a tool 201. The tool 201 is moveable and is used to apply pressure to the article 100. The tool 201 may also be heated to apply heat to the article 100. The heat may also be applied by a source separate to the tool 201 such as a separate heater of the embossing machine 200. The tool 201 has a structure surface 203. The structure surface 203 has a positive profile that corresponds to the raised profile formed in the article 100 as a result of the embossing process. The embossing machine 200 further comprise a mould component 205 with a chamber 207. The chamber 207 is a recessed or impressed portion of the mould component 205 that is shaped to be the negative of the raised profile to be formed in the article 100 as a result of the embossing process.

The article 100 is positioned in the embossing machine 200 such that the embossing material 105 is positioned proximate to the tool 201 and the conductive region 103 is positioned proximate to the mould component 205. This means that the tool 201 applies pressure and optionally heat to the embossing material 105 which causes the article 100 to distort and urges the conductive region 103 into the chamber 207 of the mould component 205.

The tool 201 may be a ram or piston. The embossing machine 200 may be a piston-and-chamber mould. The embossing machine 200 may be a heat press. The embossing machine 200 may be a roller embosser. The embossing machine 200 may be an ultrasonic embosser. Machines for embossing articles and especially textile articles are well-known to the skilled person. Such embossing machines 200 are commonly used to form raised graphical profiles on garments for decorative purposes. It will be apparent to the skilled person that any form of embossing machine 200 suitable for forming the articles 100 described herein will be suitable for implementing the claimed invention.

The tool 201 may have a structured surface 203 that has positive and negative regions. That is the structured surface 203 may include regions that project outwardly and regions that are recessed or impressed into the surface of the tool 201. The mould component 205 may also comprise positive and negative regions. The use of positive and negative regions in the tool 201/mould component 205 may result in raised and impressed regions being formed on a surface of the article 100.

Figure 3 shows that the tool 201 is moved towards the embossing material 105 in the direction of the arrow 209 so as to apply pressure and optionally heat to the article 100.

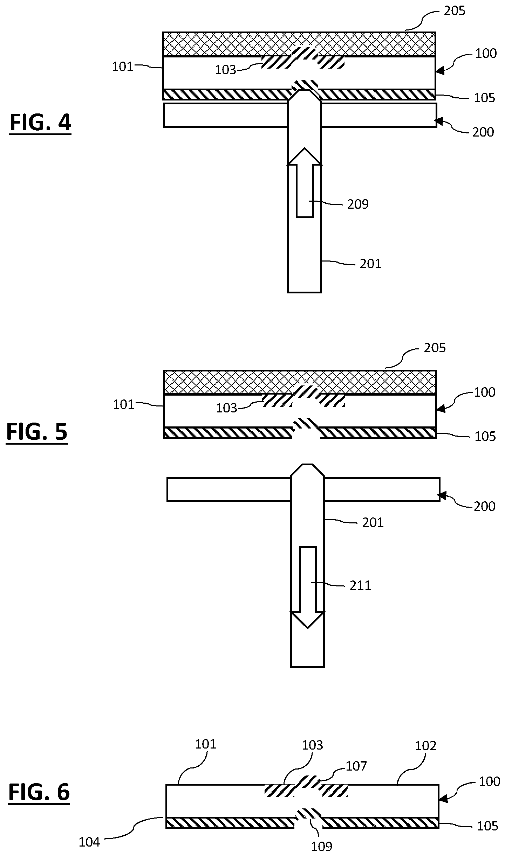

Figure 4 shows that the compressive force applied by the tool 201 causes the article 100 to distort. In particular, the tool 201 pushes the embossing material 105, textile body 101 and conductive region 103 in the direction of the arrow 209. This forces the conductive region 103 into the mould cavity 207. The pressure and optionally heat applied by the tool 201 and optionally other components of the embossing machine 200 activates the embossing material 105 and causes the embossing material 105 to bond with the textile body 101.

Figure 5 shows that after the tool 201 is retracted away from the article 100 in the direction of arrow 211 the article 100 retains its distorted shape upon release of the applied pressure. That is, the embossing material 105 bonds to the article 100 so as to cause the article 100 to permanently retain its embossed profile.

Figure 6 shows the article 100 after it has been removed from the embossing machine 200. The conductive region 103 has a raised region 107 that extends away from the first surface 102 of the textile body 101. The conductive region 103 has a raised, embossed, profile 107 and forms a three-dimensional conductive region1 03. Only a part of the conductive region 103 is raised in this example but the whole of the conductive region 103 may be raised if desired. In addition, parts of the textile body 101 may also be raised if desired. The size and shape of the raised profile formed in the conductive region 103/textile body 101 can be varied by appropriately modifying the tool 201 and mould component 205.

The embossing material 105 causes the conductive region 103 to permanently retain the raised, embossed, profile 107 and provides stability to the raised, embossed, profile 107. The raised profile 107 may be considered as an embossed zone 107 in the textile article 100. The embossed zone 107 comprises conductive material. A recess 109 that corresponds to and is aligned with the embossed region 107 is formed in the vicinity of the second surface 104 the textile body 101.

Advantageously, the present disclosure provides an article 100 with a raised conductive region 103, 107 that projects outwardly from a main surface 102 of the textile body 101. The raised conductive region 103 is beneficial in enhancing signal contact with a skin surface such as when the conductive region 103 is used as an electrode. There are other beneficial applications for raised conductive regions 103 besides electrodes that form a signal coupling with a skin surface. The approach of the present disclosure means that the raised conductive region 103 is formed without requiring the dense packing of the space between the conductive region 103 and the textile body 101 using a filler yarn or other filler/support material. Instead, established embossing techniques which are commonly used to form raised decorative patterns on fabrics are used. To the inventor's knowledge embossing techniques have not previously been used to form raised conductive regions. The approach of the present disclosure simplifies manufacture of the article 100 as less complicated machinery and yarns are required. In addition, waterproofing properties are provided by the embossing material 105. A separate process for waterproofing the article 100 is therefore not required.

The article 100 may form or be otherwise incorporated into a wearable article such as a garment although this is not required. The article 100 may form part of the fabric of the wearable article/garment. The article 100 may be a stand-alone article or may be incorporated into other forms of textiles/fabrics and furnishings such as textile/fabric covers and upholstery. The article 100 may comprise knitted, woven or felted material and generally comprise fabrics that are knitted or woven.

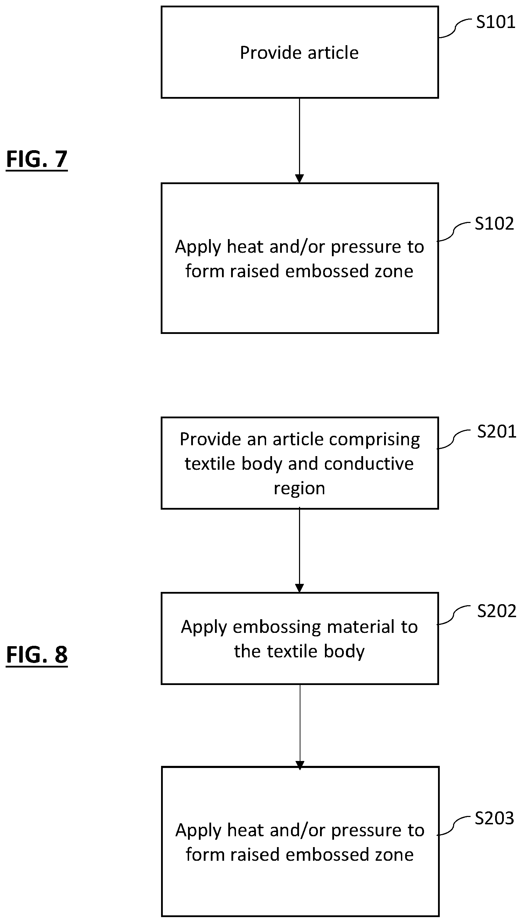

Referring to Figure 7, there is shown a process flow diagram for an example method of forming a raised profile in a conductive region of an article according to aspects of the present disclosure. Step S101 of the method comprises providing an article.

Step S102 of the method comprises applying heat and/or pressure to the article to cause the article to adopt a raised, embossed, profile that projects outwardly from a surface of a textile body of the article. The raised, embossed, profile is retained upon release of the applied heat and/or pressure due to an embossing material of the article bonding to the textile body due to the heat and/or pressure. The raised, embossed profile is a conductive region of the article.

Referring to Figure 8, there is shown a process flow diagram for an example method of forming a raised profile in a conductive region of an article according to aspects of the present disclosure. Step S201 of the method comprises providing an article comprising a textile body and a conductive region.

Step S202 of the method comprises applying an embossing material to the textile body.

Step S203 comprises applying heat and/or pressure to the article to cause the article to adopt a raised, embossed, profile that projects outwardly from a surface of a textile body of the article. The raised, embossed, profile is retained upon release of the applied heat and/or pressure due to an embossing material of the article bonding to the textile body due to the heat and/or pressure. The raised, embossed profile is a conductive region of the article.

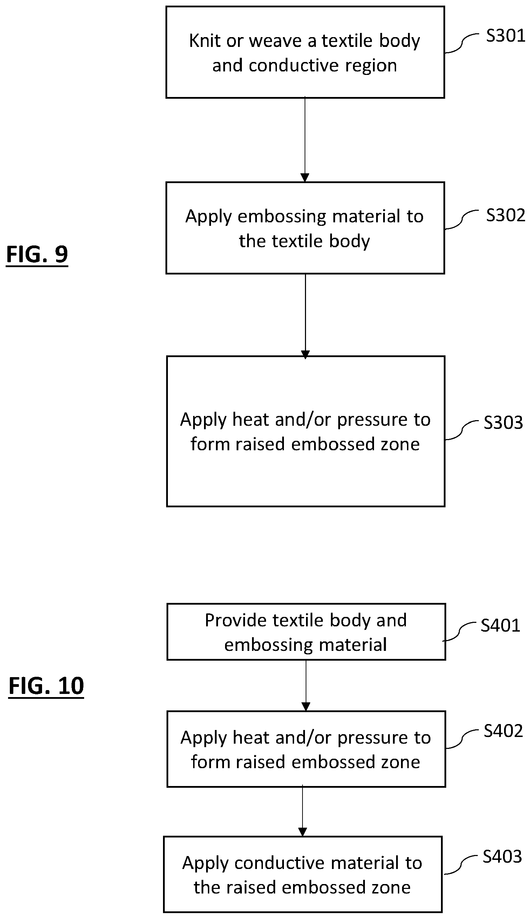

Referring to Figure 9, there is shown a process flow diagram for an example method of forming a raised profile in a conductive region of an article according to aspects of the present disclosure.

Step S301 of the method comprises knitting or weaving a textile body and conductive region of an article.

Step S302 of the method comprises applying an embossing material to the textile body.

Step S303 comprises applying heat and/or pressure to the article to cause the article to adopt a raised, embossed, profile that projects outwardly from a surface of a textile body of the article. The raised, embossed, profile is retained upon release of the applied heat and/or pressure due to an embossing material of the article bonding to the textile body due to the heat and/or pressure. The raised, embossed profile is a conductive region of the article.

Referring to Figure 10, there is shown a process flow diagram for an example method of forming a raised profile in a conductive region of an article according to aspects of the present disclosure.

Step S401 of the method providing an article comprising a textile body and an embossing material.

Step S402 comprises applying heat and/or pressure to the article to cause the article to adopt a raised, embossed, profile that projects outwardly from a surface of a textile body of the article. The raised, embossed, profile is retained upon release of the applied heat and/or pressure due to an embossing material of the article bonding to the textile body due to the heat and/or pressure.

Step S403 comprises applying conductive material to the raised, embossed, profile of the article to form a raised conductive region. The conductive material may be printed, transferred, or otherwise deposited onto the textile body.

In the above example methods, the embossing material may be printed onto the textile body or applied as a film onto the textile body. Other methods of applying the embossing material to the textile body are within the scope of the present disclosure. The embossing material may be in the form of an embossing (thermoformable) yarn that is incorporated into the article.

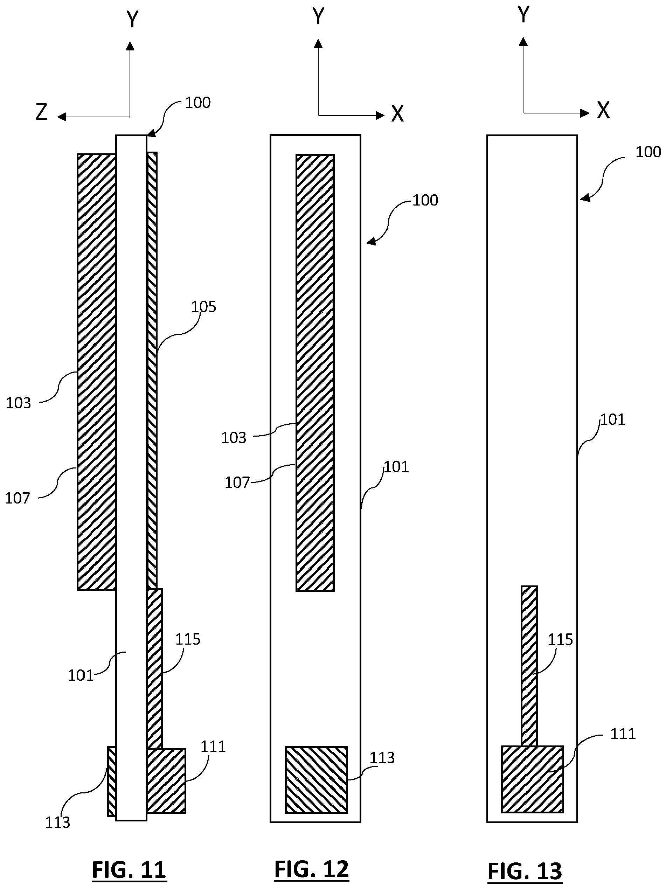

In the above example methods, applying heat and/or pressure to the article may comprise providing a tool; and using the tool to apply pressure to the article to adopt the raised embossed, profile. Applying heat and/or pressure to the article may further comprise providing a mould component having a cavity and may comprise using the tool to apply pressure to the article may distort the conductive region into the cavity of the mould component so as to adopt the raised profile. The tool may have a structured surface. The structure surface may comprise a positive profile that corresponds to the raised profile to be formed in the conductive region or a negative profile that is the inverse of the raised profile to be formed in the conductive region. Referring to Figures 11 to 13, there is shown an example article 100 according to aspects of the present disclosure.

The article 100 is an elongate and narrow strip of material. The article 100 is able to be worn so as to obtain measurement signals from the wearer. The article 100 may be used to form a chest strap or wrist strap or may be integrated into a separate wearable article such as a garment. The article 100 may be adhesively bonded to an inner surface of a garment for example.

The article 100 comprises a continuous body of fabric. Here, continuous body of fabric, refers to a unitary fabric structure that may be integrally knit, woven or felted. Seams are not provided between different sections of the continuous body of fabric. In other words, the fabric is seamless. Although the fabric is seamless, different types of yarns such as conductive and non-conductive yarns are provided in the continuous body of fabric. The body of fabric in this example is a knitted fabric and, in particular, a weft knitted fabric.

The continuous body of fabric 100 comprises a double-knit non-conductive textile body 101. The double-knit non-conductive textile body 101 comprises first and second interconnected knit layers. The first knit layer defines first surface and the second knit layer defines second surface opposing the first surface. The first surface and the second surface are parallel to one another and spaced apart along the Z axis. In use, the first surface faces towards the skin surface of the wearer of the article 100 and the second surface faces away from the skin surface of the wearer. The first surface may be referred to as the back/inner surface and the second surface may be referred to as the front/outer surface. The use of a double-knit structure is not required. The present disclosure is not limited to such examples. The textile body 101 may have a single bed structure, a links structure, or a ribbed structure for example.

The non-conductive textile body 101 is formed from a non-conductive base yarn. In this example, the non-conductive base yarn is a composite elastomeric yarn. In particular, a composite elastomeric yarn comprising 81% nylon and 19% elastane is used. Of course, other non-conductive yarns may be used as desired by the skilled person.

The non-conductive textile body 101 may comprise additional yarns which may be incorporated during the knitting of the textile body 101. In this example, the textile body 101 further comprises additional elastomeric yarn to provide additional stretch in the textile body 101. This may improve the comfort of the article 100 and help ensure that an electrode of the article 100 is help in contact with the skin surface. In this example, elastomeric yarn number 815 by Stretchline Limited is used. The additional elastomeric yarn may not be required if, for example, a high degree of stretch is not desired or the base textile yarn already as the desired degree of stretch.

In this example, the textile body 101 further comprises a sealing/bonding yarn to seal the edges of the article 100 to reduce and even prevent fraying of the textile article. An example sealing/bonding yarn is the Porte yarn from Nittobo Group of Japan. The present disclosure is not limited to this example, and other sealing/bonding yarns are within the scope of the present disclosure.

The article 100 further comprises a sensing component that comprises a first conductive region 103, second conductive region 111 and conductive pathway 115 extending between the first and second conductive regions 103, 111. The sensing component is integrally formed with the textile body 101. The sensing component is formed from conductive yarn, and in particularly is a unitary knitted structure formed from a single length of conductive yarn. This means that separate wires, connectors or other hardware elements are not required to electrically connect the different parts of the sensing component together. In this example, Circuitex ^{™} conductive yarn from Noble Biomaterials Limited is used to form the sensing component. Of course, other conductive yarns may be used. The conductive yarn may comprise a non-conductive or less conductive base yarn which is coated or embedded with conductive material such as carbon, copper and silver.

The sensing component comprises a first conductive region 103. The first conductive region 103 is provided on the first surface and extends along part of the length of the article 100 in the direction of the Y-axis. The first conductive region 103 is a three-dimensional conductive region 103 that extends away from the first surface along the Z-axis. This three-dimensional/raised conductive region 103 forms a three-dimensional/raised electrode 103 for contacting the skin surface of the wearer to measure signals from the wearer and/or introduce signals into the wearer. The first conductive region 103 comprises a plurality of courses of conductive yarn. Opposing end courses of the conductive yarn are interconnected with the knit layer defining the first surface of textile body 101. The remaining courses of conductive yarn extend away from the first surface of the textile body 101 to form the raised conductive region. The raised profile 107 of the conductive region 103 is maintained as a result of the embossing material 105 provided on the second surface of the textile body 101. Having a raised conductive region 103 is beneficial for improving electrode contact with the skin surface particularly when the wearer is moving.

The first conductive region 103/electrode 103 may be arranged to measure one or more biosignals of a user wearing the article 100. Here, "biosignal" may refer to any signal in a living being that can be measured and monitored. The electrode 103 is generally for performing bioelectrical or bioimpedance measurements. Bioelectrical measurements include electrocardiograms (ECG), electrogastrograms (EGG), electroencephalograms (EEG), and electromyography (EMG). Bioimpedance measurements include plethysmography (e.g., for respiration), body composition (e.g., hydration, fat, etc.), and electroimpedance tomography (EIT). The electrode 103 may additionally or separately be used to apply an electrical signal to the wearer. This may be used in medical treatment or therapy applications.

The sensing component further comprises a second conductive region 111. The second conductive region 111 is provided on the second surface of the textile body 101 and extends along part of the length of the article 100 along the Y-axis. The second conductive region 111 is a three-dimensional conductive region 111 that extends away from the second surface along the Z axis. The second conductive region 111 forms a connectional terminal 111 for electrically connecting with an electronics module as explained in greater detail below. The second conductive region 111 comprises a plurality of courses of conductive yarn. Opposing end courses of the conductive yarn are interconnected with the knit layer defining the second surface of the base textile body 101. The remaining courses of conductive yarn extend away from the second surface of the textile body 101 to form the raised conductive region 111. The raised profile of the conductive region 111 is maintained as a result of the embossing material 113 provided on the first surface of the textile body 101. Having a raised connection terminal 111 is beneficial in terms of improving the electrical connection between the connection terminal 111 and the electronics module.

The first conductive region 103 and the second conductive region 111 are spaced apart from one another along the length of the article 100. That is, they are spaced apart along the Y-axis.

The sensing component further comprises a conductive pathway 115. The conductive pathway 115 extends along the length of the article 100 between the first conductive region 103 and the second conductive region 111 to electrically connect the first conductive region 103 to the second conductive region 111. The conductive pathway 115 is substantially flush with the second surface of the textile body 101 and is formed from one or more (two in this example) of courses of conductive yarn extending between adjacent courses of non-conductive yarn in the textile body 101. Proximate to the first conductive region 103, part of the conductive yarn extends through the textile body 101 so as enable the conductive pathway 115 to be formed on the second surface of textile body 101 while still being electrically connected to the first conductive region 103. The conductive pathway 115 does not form a raised region and is substantially flush with the textile body 101. An embossing material is not provided in the vicinity of the conductive pathway 115 in this example and the conductive pathway 115 does not thus form an embossed zone in the article. An embossing material may still be provided to overlap the conductive pathway 115 but may not be moulded to form an embossed zone. In some examples, the embossing material 105 may cover all or part of the conductive pathway so as to protect the conductive pathway and provide waterproofing properties.

The textile body and sensing component can be manufactured integrally in a single knitting operation. This means that discrete electronic components do not need to be integrated into an already formed textile body but instead the sensing component is formed of conductive yarn as the textile body is being knitted. The resultant article has a singular textile/fabric structure which handles, feels, behaves and looks like a fabric while providing the desired sensing functionality.

The textile body 101 and conductive regions 103, 111, 115 are made using a flat-bed knitting machine that has a front bed of needles and a back bed of needles. Additional beds of needles may be provided and used in the knitting process. Other knitting machines capable such as circular knitting machines may also be used to manufacture the article 100 generally the knitting machines are required to have at least first and second beds of needles.

The electrode 103 is wider along the X axis than the conductive pathway 115. Having a wider electrode 103 is beneficial in providing increased surface area of electrode 103 contact with the skin surface. Having a narrower conductive pathway 115 is beneficial in terms of improving comfort for the wearer and minimising the visual appearance of the sensing component on the article 100. The connection terminal 111 is also wider along the X axis than the conductive pathway 115. Having a wider connection terminal 111 is beneficial in terms of improving the electrical connection between the connection terminal 111 and the electronics module.

The construction of article 100 in Figures 11 to 13 provides the electrode 103 and connection terminal 111 on opposed surfaces of the textile body 101. This is not required in all examples of the present disclosure as, in some examples, the electrode 103 and the connection terminal 111 may be provided on the same surface of the textile body 101. However, the arrangement of Figures 11 to 13 is preferred as it enables an electronics module to be connected to the electrode 103 from the second, outer surface without additional modification to the article 100.

If the connection terminal and the electrode were both located on a first surface then additional manufacturing steps may be required to enable an electronics module located on the second surface to extend through the hole to connect with the connection terminal. For example, a hole may have to be formed in the article. Forming the hole may require additional manufacturing steps which may increase the time and cost of manufacturing the textile article. Moreover, the hole may weaken the structural integrity of the article. In another example, a conductive fastener such as a conductive metal stud may be inserted into the textile body to allow the interface element to connect with the connection terminal on the first surface. Incorporating additional hardware into the textile article may increase the manufacturing costs and reduce the comfort and visual appearance of the textile article.

In this example, the textile body 101 and conductive regions 103, 111, 115 are integrally knit during a knitting operation. The embossing materials 105, 113 are then applied to the textile body 101. The article 100 is then positioned in an embossing machine 200 to cause the raised profiles to be permanently formed in the article 100. This approach means that an isolating thread network/filler yarn does not need to be deposited in a space formed between the textile body 101 and the conductive regions 103, 111. This simplifies the knitting operations and means that complicated knitting techniques and specialist yarns are not required to form the article 100. Instead, known embossing techniques which are established techniques used in textile processing can be used to form the raised conductive profiles 103, 111.

The article 100 may be attached to a wearable article such as a garment. The article 100 may be integrally knit with the wearable article. Such as by integrally knitting a garment comprising the article 100.

The present disclosure is not limited to any particular dimension of the electrode 103, conductive pathway 115, and connection terminal 111. Generally, however, the electrode 103, the conductive pathway 115, and connection terminal 111 extend for a height of between 0.2mm and 50mm along the Z-axis.

The electrode 103, conductive pathway 115, and connection terminal 111 extend for a width of at least 0.1 mm along the X axis. The electrode 103 and/or connection terminal 111 and/or conductive pathway 115 may extend for a width of at least 0.5 mm, at least 1 mm, at least 2 mm, or at least 3 mm. The electrode 103 and/or connection terminal 111 may have a width of at least 3 mm, at least 5 mm, at least 10 mm, at least 15 mm, at least 20 mm, or at least 50 mm. The electrode 103 and/or connection terminal 111 may have a width between 5 mm and 20 mm.

The electrode 103, conductive pathway 115, and connection terminal 111 extend for a length of at least 1 mm along the Y axis. The electrode 103 may have a length of at least 5 mm, at least 10 mm, at least 20 mm, at least 50 mm, or at least 100 mm. The electrode 103 may have a length of between 20 and 50 mm. The connection terminal 111 may have a length of at least 5 mm, at least 10 mm, at least 20 mm, at least 50 mm, or at least 100 mm. The connection terminal 111 may have a length of between 5 mm and 10 mm. The conductive pathway 115 may extend for a least of at least 5 mm, at least 10 mm, at least 20 mm, at least 50 mm, at least 100 mm, at least 200 mm, at least 300 mm, at least 500 mm. The conductive pathway 115 may extend for a length of the between 100 mm and 300mm.

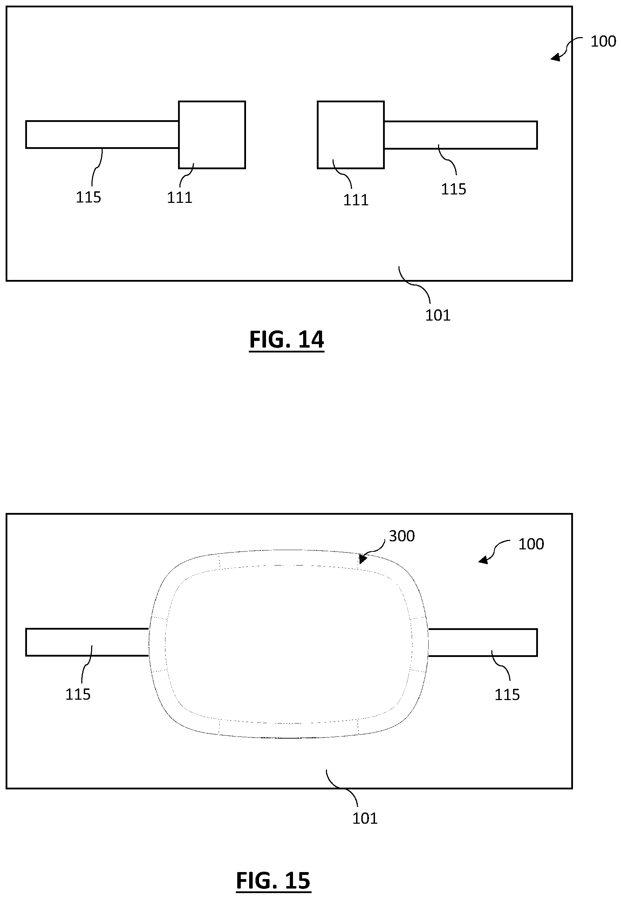

Referring to Figures 14 to 17, there is shown how an electronics module 300 may be positioned on an article 100 so that the electronics module 300 may form a communicative connection with the conductive regions 103, 111 of the article 100.

The article 100 in this example comprises a textile body 101. A pair of conductive regions 103 forming electrodes are provided on a first surface of the textile body 101. The conductive regions 103 have raised, embossed, profiles. A pair of conductive regions 111 are provided on a second surface of the textile body 101. The conductive regions 111 form connection regions 111 that contact with the electronics module 300 when in use. Conductive pathways 115 extend from and connect each of the connection regions 111 to a respective one of the electrodes 103.

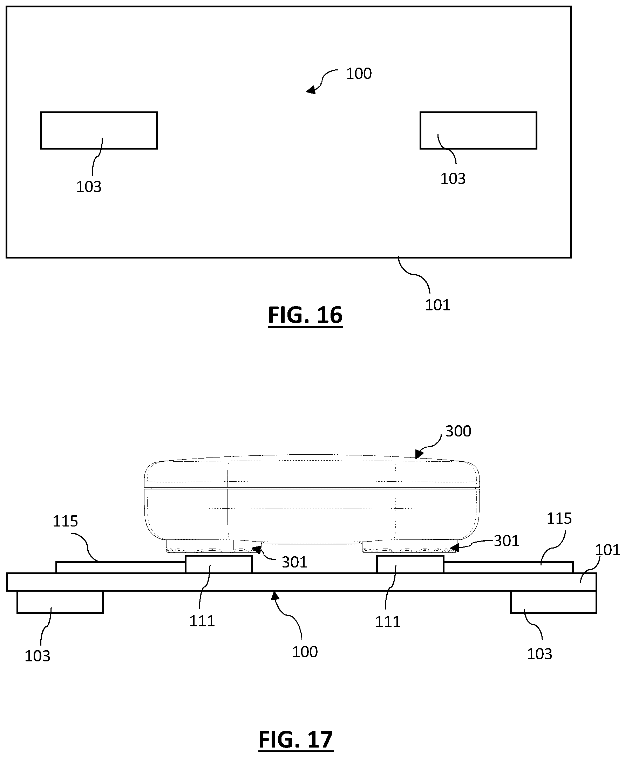

Figure 17 shows the electronics module 300 comprises interface elements 103 in the form of contact pads 301. The electronics module 300 is positioned on a surface of the textile body 101 such that the contact pads 301 contact and electrically connect with the connection regions 111. In this way, the electronics module 300 is able to receive measurement signals from the electrodes 103 via the conductive pathways 115. The signals are typically biosignals obtained when the electrodes 103 are placed in contact with a skin surface of a wearer.

The electronics module 300 comprises a housing formed of a rigid material in this example. One or more electrical components are provided within the rigid housing. The housing may comprise a (rigid) polymeric material. The polymeric material may be a rigid plastic material. The rigid plastic material may be ABS or polycarbonate plastic but is not limited to these examples. The rigid plastic material may be glass reinforced. The rigid housing may be injection moulded. The rigid housing may be constructed using a twin-shot injection moulding approach.

A plurality (two in this example) of contact pads 301 are provided on the outer surface of the housing. The contact pads 301 are formed from a flexible material, but this is not required in all examples. The contact pads 301 are spaced apart from one another on the bottom surface of the housing. "Rigid" will be understood as referring to a material which is stiffer and less able to bend than the contact pads 301 formed of flexible material. The rigid housing may still have some degree of flexibility but is less flexible than the flexible material of the contact pads 301.

The contact pads 301 comprise conductive material, and thus acts as conductive contact pads 301 for the electronics module 300. The flexible conductors 301 therefore provide the interface by which the electronics module 300 is able to receive signals from an external component such as the garment 400.

The contact pads 301 conductively connect with connection regions 111 of the article 100. Each of the contact pads 301 is conductively connected with a different one of the connection regions 111.

The use of flexible conductors 301 is generally preferred as compared to rigid, metallic, conductors 301 as this means that hard pieces of conductive metallic material such as poppers or studs are not required to electrically connect the electronics module 300 to the article 100. This not only improves the look and feel of the article 100 but also reduces manufacturing costs as it means that hardware features such as additional eyelets and studs do not need to be incorporated into the article 100 to provide the required connectivity. An additional problem with rigid metallic conductors is that their hard, abrasive, surfaces may rub against conductive elements such as conductive thread of the article 100 and cause the conductive thread to fray.

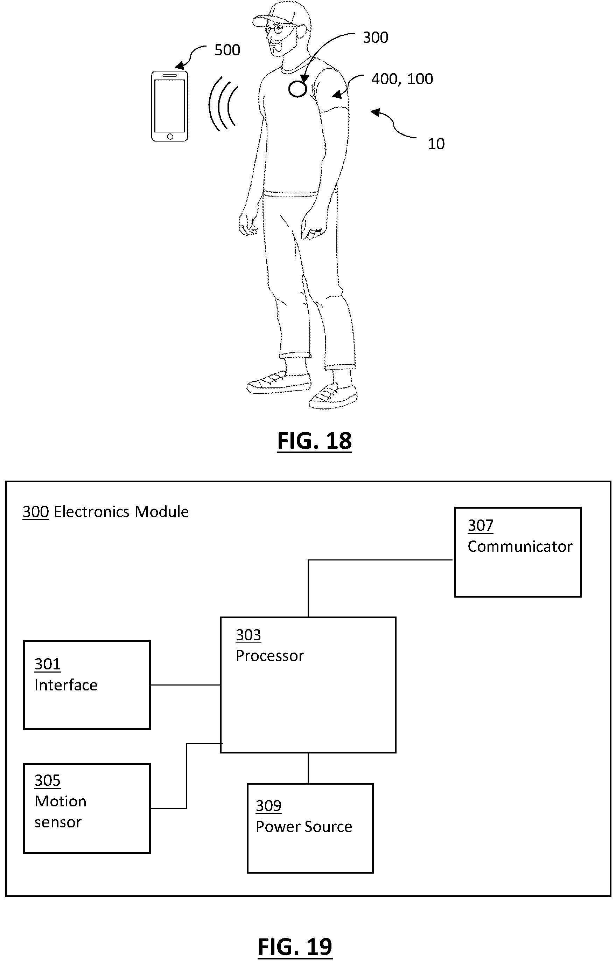

Referring to Figure 18, there is shown an example system 10 according to aspects of the present disclosure. The system 10 comprises an electronics module 300, and a garment 400. The garment 400 is formed from or incorporates the article 100 according to aspects of the present disclosure. The system 10 further comprises a mobile device 500. The garment 400 is worn by a user. The electronics module 300 is attached to the garment 400. The electronics module 300 is shown positioned on a textile body of the garment 400 in Figure 1. The electronics module 300 may be positioned within a pocket or similar mounting arrangement of the garment 400.

The electronics module 300 is arranged to integrate with sensing components incorporated into the article 100/garment 400 so as to obtain signals from the sensing components. The sensing components may comprise electrodes. The electronics module 300 is further arranged to wirelessly communicate data to the mobile device 500. Various protocols enable wireless communication between the electronics module 300 and the mobile device 500. Example communication protocols include Bluetooth ^{®}, Bluetooth ^{®} Low Energy, and near-field communication (NFC). In some examples, the electronics module 300 may communicate over a long-range wireless communication protocol.

The electronics module 300 is removable from the garment 400. The mechanical coupling of the electronic module 300 to the garment 400 may be provided by a mechanical interface such as a clip, a plug and socket arrangement, pocket etc. The mechanical interface may be referred to as an electronics module holder of the garment 400. The electronics module holder may be an elasticated pocket that applies pressure to hold the electronics module 300 in place.

Beneficially, the removable electronic module 300 may contain all of the components required for data transmission and processing such that the garment 400 only comprises the sensor components and communication pathways. In this way, manufacture of the garment 400 may be simplified. In addition, it may be easier to clean a garment 400 which has fewer electronic components attached thereto or incorporated therein. Furthermore, the removable electronic module 300 may be easier to maintain and/or troubleshoot than embedded electronics. The electronic module 300 may comprise flexible electronics such as a flexible printed circuit (FPC). The electronic module 300 may be configured to be electrically coupled to the garment 400.

It may be desirable to avoid direct contact of the electronic module 300 with the wearer's skin while the garment 400 is being worn. It may be desirable to avoid the electronic module 300 coming into contact with sweat or moisture on the wearer's skin or other sources of moisture such as from rain or a shower. It may further be desirable to provide an electronics module holder such as a pocket in the garment to contain the electronic module 300 in order to prevent chafing or rubbing and thereby improve comfort for the wearer. The pocket may be provided with a waterproof lining in order to prevent the electronic module 300 from coming into contact with moisture.

Referring to Figure 19, there is shown a schematic diagram for an example electronics module 300 according to aspects of the present disclosure.

The electronics module comprises an interface 301. The interface 301 is arranged to communicatively couple with a sensing component of the garment 400 so as to receive a signal from the sensing component or may directly interface with a skin surface of the wearer to receive signals therefrom. The interface 301 may form a conductive coupling or a wireless (e.g. inductive) communication coupling with the electronics components of the garment 400. The interface 301 may comprise the contact pads 301.

The electronics module 300 comprises a processor 303. The processor 303 is communicatively coupled to the interface 301 and is arranged to receive the signals from the interface 301. The processor 303 is configured to process signals sensed by a sensing component of the electronics module 300 and/or the garment 400. The signals relate to the activity of a user wearing the garment 400.

The electronics module 300 comprises a motion sensor 305 such as an inertial measurement unit 305. The inertial measurement unit 305 may comprise an accelerometer and optionally one or both of a gyroscope and a magnetometer. A gyroscope/magnetometer is not required in all examples, and instead only an accelerometer may be provided or a gyroscope/magnetometer may be present but put into a low power state. A processor of the sensor 305 may perform processing tasks to classify different types of detected motion. The processor of the sensor 305 may, in particular, perform machine-learning functions so as to perform this classification. Performing the processing operations on the sensor 305 rather than the processor 303 is beneficial as it reduces power consumption and leaves the processor 303 free to perform other tasks. In addition, it allows for motion events to be detected even when the processor 303 is operating in a low power mode. The sensor 305 communicates with the processor 303 over a serial protocol such as the Serial Peripheral Interface (SPI), Inter-Integrated Circuit (I2C), Controller Area Network (CAN), and Recommended Standard 232 (RS-232). Other serial protocols are within the scope of the present disclosure.

The electronics module 300 comprises a communicator 307. The communicator 307 may be a mobile/cellular communicator operable to communicate the data wirelessly via one or more base stations. The communicator 307 may provide wireless communication capabilities for the garment 400 and enables the garment 400 to communicate via one or more wireless communication protocols such as used for communication over: a wireless wide area network (WWAN), a wireless metroarea network (WMAN), a wireless local area network (WLAN), a wireless personal area network (WPAN), Bluetooth ^{®} Low Energy, Bluetooth ^{®} Mesh, Bluetooth ^{®} 5, Thread, Zigbee, IEEE 802.15.4, Ant, a near field communication (NFC), a Global Navigation Satellite System (GNSS), a cellular communication network, or any other electromagnetic RF communication protocol. The cellular communication network may be a fourth generation (4G) LTE, LTE Advanced (LTE-A), LTE Cat-M1, LTE Cat-M2, NB-loT, fifth generation (5G), sixth generation (6G), and/or any other present or future developed cellular wireless network. A plurality of communicators may be provided for communicating over a combination of different communication protocols.

The electronics module 300 may comprise a Universal Integrated Circuit Card (UICC) that enables the electronics module 300 to access services provided by a mobile network operator (MNO) or virtual mobile network operator (VMNO). The UICC may include at least a read-only memory (ROM) configured to store an MNO/VMNO profile that the wearable article can utilize to register and interact with an MNO/VMNO. The UICC may be in the form of a Subscriber Identity Module (SIM) card. The electronics module 300 may have a receiving section arranged to receive the SIM card. In other examples, the UICC is embedded directly into a controller of the electronics module 300. That is, the UICC may be an electronic/embedded UICC (eUICC). A eUICC is beneficial as it removes the need to store a number of MNO profiles, i.e. electronic Subscriber Identity Modules (eSIMs). Moreover, eSIMs can be remotely provisioned to electronics modules 300. The electronics modules 100 may comprise a secure element that represents an embedded Universal Integrated Circuit Card (eUICC).

The electronics module 300 comprises a power source 309. The power source 309 is coupled to the processor 303 and is arranged to supply power to the processor 303. The power source 309 may comprise a plurality of power sources. The power source 309 may be a battery. The battery may be a rechargeable battery. The battery may be a rechargeable battery adapted to be charged wirelessly such as by inductive charging. The power source 309 may comprise an energy harvesting device. The energy harvesting device may be configured to generate electric power signals in response to kinetic events such as kinetic events performed by a wearer of the garment. The kinetic event could include walking, running, exercising or respiration of the wearer. The energy harvesting material may comprise a piezoelectric material which generates electricity in response to mechanical deformation of the converter. The energy harvesting device may harvest energy from body heat of a wearer of the garment. The energy harvesting device may be a thermoelectric energy harvesting device. The power source may be a super capacitor, or an energy cell.

The electronics module 300 is mounted on a garment 400 and conductively connected to sensing components such as electrodes of the garment via electrically conductive pathways of the garment 400. In a particular example, the sensing components are electrodes used to measure electro potential signals such as electrocardiogram (ECG) signals.

In summary, there is provided an article and a method of making the same. The article 100 comprises a textile body 101, a conductive region 103 and an embossing material 105. The embossing material 105 causes the conductive region 103 to adopt and retain a raised, embossed, profile 107 that projects outwardly from a surface 102 of the textile body 101.The method comprises applying heat and/or pressure to the article 100 to cause the article 100 to adopt the embossed profile 107. The raised, embossed, profile 107 is retained upon release of the applied heat and/or pressure as the embossing material 105 has bonded to the textile body 101 due to the application of heat and/or pressure.

In the present disclosure, the electronics module may also be referred to as an electronics device or unit. These terms may be used interchangeably.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA), programmable System on Chip (pSoC), or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables.

## Claims

1. An article (100) comprising a textile body (101), a conductive region (103, 111) that forms an electrode arranged to measure or apply signals to a further object, and an embossing material (105, 113), wherein the embossing material (105, 113) causes the conductive region (103, 111) to adopt and retain a raised, embossed, profile (107) that projects outwardly from a surface of the textile body (101), **characterised in that** the embossing material (105, 113) comprises a heat and/or pressure-activated adhesive material.

2. An article (100) as claimed in claim 1, wherein the embossing material (105, 113) is applied to a surface (102, 104) of the textile body (101).

3. An article (100) as claimed in claim 2, wherein the embossing material (105) is applied to a surface (104) of the textile body (101) that opposes a surface (102) on which the conductive region (103) is located.

4. An article (100) as claimed in any preceding claims, wherein the embossing material (105) is waterproof.

5. An article (100) as claimed in any preceding claim, wherein the embossing material (105) comprises silicone.

6. An article (100) as claimed in any preceding claim, wherein the embossing material (105) comprises an embossing ink.

7. An article (100) as claimed in any preceding claim, wherein the embossing material (105) comprises an embossing film.

8. An article (100) as claimed in any preceding claim, wherein the embossing material (105) comprises an embossing yarn.

9. An article (100) as claimed in any preceding claim, wherein the conductive region (103) comprises conductive yarn.

10. An article (100) as claimed in claim 9, wherein the conductive region (103) is integrally formed with the textile body (101).

11. An article (100) as claimed in any preceding claim, wherein the article (100) comprises a plurality of embossed conductive regions (103).

12. A method of forming a raised profile (107) in a conductive region (103, 111) of an an article (100), wherein the conductive region (103, 111) forms an electrode arranged to measure or apply signals to a further object **characterised in that** the method comprises applying heat and/or pressure (S102, S203, S303, S402) to an article (100) to cause the article (100) to adopt a raised, embossed, profile (107) that projects outwardly from a surface of a textile body (101) of the article (100), wherein the raised, embossed, profile (107) is retained upon release of the applied heat and/or pressure due to an embossing material (105, 113) of the article (100) bonding to the textile body (101) following the application of heat and/or pressure, and wherein the raised, embossed profile (107) is a conductive region (103, 111) of the aricle (100).

13. A method as claimed in claim 12, wherein applying heat and/or pressure (S102, S203, S303, S402) to the article (100) comprises providing a tool (201); and using the tool (201) to apply pressure to the article (100) to cause the article (100) to adopt the raised, embossed, profile. profile (107).

14. A method as claimed in claim 13, further comprising providing a mould component (203) having a cavity; and optionally using the tool (201) to apply pressure to the article (100) to distort the article (100) into the cavity of the mould component (203) so as to cause the article (100) to adopt the raised profile (107).

15. A method as claimed in claim 14, wherein the tool (201) has a structured surface, the structured surface having a positive profile that corresponds to the raised profile (107) to be formed in the article (100) or a negative profile that is the inverse of the raised profile (107) to be formed in the article. (100).

## Patentansprüche

1. Artikel (100), umfassend einen Textilkörper (101), einen leitfähigen Bereich (103, 111), der eine Elektrode bildet, die angeordnet ist, um Signale zu messen oder auf einen weiteren Gegenstand zu übertragen, und ein Prägematerial (105, 113), wobei das Prägematerial (105, 113) bewirkt, dass der leitfähige Bereich (103, 111) ein erhöhtes, geprägtes Profil (107) annimmt und behält, das nach außen aus einer Oberfläche des Textilkörpers (101) herausragt, **dadurch gekennzeichnet, dass** das Prägematerial (105, 113) ein durch Wärme und/oder durch Druck aktiviertes Klebematerial umfasst.

2. Artikel (100) nach Anspruch 1, wobei das Prägematerial (105, 113) auf eine Oberfläche (102, 104) des Textilkörpers (101) aufgebracht ist.

3. Artikel (100) nach Anspruch 2, wobei das Prägematerial (105) auf eine Oberfläche (104) des Textilkörpers (101) aufgebracht ist, die einer Oberfläche (102) gegenüberliegt, auf der sich der leitfähige Bereich (103) befindet.

4. Artikel (100) nach einem der vorhergehenden Ansprüche, wobei das Prägematerial (105) wasserdicht ist.

5. Artikel (100) nach einem der vorhergehenden Ansprüche, wobei das Prägematerial (105) Silikon umfasst.

6. Artikel (100) nach einem der vorhergehenden Ansprüche, wobei das Prägematerial (105) eine Prägefarbe umfasst.

7. Artikel (100) nach einem der vorhergehenden Ansprüche, wobei das Prägematerial (105) eine Prägefolie umfasst.

8. Artikel (100) nach einem der vorhergehenden Ansprüche, wobei das Prägematerial (105) ein Prägegarn umfasst.

9. Artikel (100) nach einem der vorhergehenden Ansprüche, wobei der leitfähige Bereich (103) leitfähiges Garn umfasst.

10. Artikel (100) nach Anspruch 9, wobei der leitfähige Bereich (103) mit dem Textilkörper (101) integral ausgebildet ist.

11. Artikel (100) nach einem der vorhergehenden Ansprüche, wobei der Artikel (100) mehrere geprägte leitfähige Bereiche (103) umfasst.

12. Verfahren zum Ausbilden eines erhöhten Profils (107) in einem leitfähigen Bereich (103, 111) eines Artikels (100), wobei der leitfähige Bereich (103, 111) eine Elektrode bildet, die angeordnet ist, um Signale zu messen oder auf einen weiteren Gegenstand zu übertragen, **dadurch gekennzeichnet, dass** das Verfahren das Anwenden von Wärme und/oder Druck (S102, S203, S303, S402) auf einen Gegenstand (100) umfasst, um zu bewirken, dass der Gegenstand (100) ein erhöhtes, geprägtes, Profil (107) annimmt, das von einer Oberfläche eines Textilkörpers (101) des Artikels (100) nach außen herausragt, wobei das erhöhte, geprägte Profil (107) bei Freigabe der angewandten Wärme und/oder des Drucks erhalten bleibt, da sich ein Prägematerial (105, 113) des Artikels (100) nach der Anwendung von Wärme und/oder Druck mit dem Textilkörper (101) verbindet,
und wobei das erhöhte, geprägte Profil (107) ein leitfähiger Bereich (103, 111) des Artikels (100) ist.

13. Verfahren nach Anspruch 12, wobei Anwenden von Wärme und/oder Druck (S102, S203, S303, S402) auf den Artikel (100) Folgendes umfasst: Bereitstellen eines Werkzeugs (201); und Verwenden des Werkzeugs (201), um Druck auf den Artikel (100) auszuüben, damit der Artikel (100) das erhöhte, geprägte Profil annimmt. Profil (107).

14. Verfahren nach Anspruch 13, ferner umfassend Bereitstellen einer Formteilkomponente (203) mit einem Hohlraum; und optional Verwenden des Werkzeugs (201) zum Anwenden von Druck auf den Artikel (100), um den Artikel (100) in den Hohlraum der Formteilkomponente (203) zu verbiegen, sodass der Artikel (100) das erhöhte Profil (107) annimmt.

15. Verfahren nach Anspruch 14, wobei das Werkzeug (201) eine strukturierte Oberfläche aufweist, wobei die strukturierte Oberfläche ein positives Profil, das dem in dem Artikel (100) auszubildenden erhöhten Profil (107) entspricht, oder ein negatives Profil, das das Inverse des in dem Artikel auszubildenden erhöhten Profils (107) ist, aufweist. (100).

## Revendications

1. Article (100) comprenant un corps textile (101), une région conductrice (103, 111) qui forme une électrode agencée pour mesurer ou appliquer des signaux sur un objet supplémentaire, et un matériau de gaufrage (105, 113), dans lequel le matériau de gaufrage (105, 113) fait en sorte que la région conductrice (103, 111) adopte et maintienne un profil relevé gaufré (107) qui fait saillie vers l'extérieur à partir d'une surface du corps textile (101), **caractérisé en ce que** le matériau de gaufrage (105, 113) comprend un matériau adhésif activé par chaleur et/ou pression.

2. Article (100) tel que revendiqué dans la revendication 1, dans lequel le matériau de gaufrage (105, 113) est appliqué sur une surface (102, 104) du corps textile (101).

3. Article (100) tel que revendiqué dans la revendication 2, dans lequel le matériau de gaufrage (105) est appliqué sur une surface (104) du corps textile (101) qui est opposée à une surface (102) sur laquelle la région conductrice (103) est située.

4. Article (100) tel que revendiqué dans une quelconque revendication précédentes, dans lequel le matériau de gaufrage (105) est étanche à l'eau.

5. Article (100) tel que revendiqué dans une quelconque revendication précédente, dans lequel le matériau de gaufrage (105) comprend de la silicone.

6. Article (100) tel que revendiqué dans une quelconque revendication précédente, dans lequel le matériau de gaufrage (105) comprend une encre de gaufrage.

7. Article (100) tel que revendiqué dans une quelconque revendication précédente, dans lequel le matériau de gaufrage (105) comprend un film de gaufrage.

8. Article (100) tel que revendiqué dans une quelconque revendication précédente, dans lequel le matériau de gaufrage (105) comprend un fil de gaufrage.

9. Article (100) tel que revendiqué dans une quelconque revendication précédente, dans lequel la région conductrice (103) comprend un fil conducteur.

10. Article (100) tel que revendiqué dans la revendication 9, dans lequel la région conductrice (103) est formée de façon monobloc avec le corps textile (101).

11. Article (100) tel que revendiqué dans une quelconque revendication précédente, dans lequel l'article (100) comprend une pluralité de régions conductrices gaufrées (103).

12. Procédé pour former un profil relevé (107) dans une région conductrice (103, 111) d'un article (100), dans lequel la région conductrice (103, 111) forme une électrode agencée pour mesurer ou appliquer des signaux sur un objet supplémentaire, **caractérisé en ce que** le procédé comprend l'application de chaleur et/ou de pression (S102, S203, S303, S402) sur un article (100) pour faire en sorte que l'article (100) adopte un profil relevé gaufré (107) qui fait saillie vers l'extérieur à partir d'une surface d'un corps textile (101) de l'article (100), dans lequel le profil relevé gaufré (107) est maintenu à la suite du retrait de la chaleur et/ou de la pression appliquée(e) en raison de la liaison d'un matériau de gaufrage (105, 113) de l'article (100) au corps textile (101) suivant l'application de chaleur et/ou de pression,
et dans lequel le profil relevé gaufré (107) est une région conductrice (103, 111) de l'article (100).

13. Procédé tel que revendiqué dans la revendication 12, dans lequel l'application de chaleur et/ou de pression (S102, S203, S303, S402) sur l'article (100) comprend la fourniture d'un outil (201) ; et l'utilisation de l'outil (201) pour appliquer la pression sur l'article (100) pour faire en sorte que l'article (100) adopte le profil relevé gaufré, profil (107).

14. Procédé tel que revendiqué dans la revendication 13, comprenant en outre la fourniture d'un composant de moule (203) ayant une cavité ; et optionnellement l'utilisation de l'outil (201) pour appliquer la pression sur l'article (100) pour déformer l'article (100) dans la cavité du composant de moule (203) afin de faire en sorte que l'article (100) adopte le profil relevé (107).

15. Procédé tel que revendiqué dans la revendication 14, dans lequel l'outil (201) a une surface structurée, la surface structurée ayant un profil positif, qui correspond au profil relevé (107), destiné à être formé dans l'article (100) ou un profil négatif, qui est l'inverse du profil relevé (107), destiné à être formé dans l'article (100).
